(19) **Europäisches Patentamt / European Patent Office / Office européen des brevets**

(11) **EP 4 765 159 A1**

(12) # EUROPEAN PATENT APPLICATION

(43) Date of publication:
24.06.2026   Bulletin 2026/26

(21) Application number: 24307202.2

(22) Date of filing: 19.12.2024

(51) International Patent Classification (IPC):
*G16H 50/70* (2018.01)      *G16H 50/80* (2018.01)

(52) Cooperative Patent Classification (CPC):
**G16H 50/80; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: ATOS France
95870 Bezons (FR)

(72) Inventors:
• LUO, Bo
  Sheffield, S10 3FP (GB)
• HNATIUK, Sam
  Knebworth, SG3 6NN (GB)
• AGENMONMEN, Philip
  Aberdeen, AB10 7GB (GB)

(74) Representative: Novagraaf Technologies
2 rue Sarah Bernhardt
CS90017
92665 Asnières-sur-Seine Cedex (FR)

(54) **COMPUTER IMPLEMENTED METHOD AND SYSTEM OF TRAINING A MACHINE LEARNING MODEL TO PREDICT AN EPIDEMIC OUTBREAK IN A GEOGRAPHICAL AREA**

(57)   A computer implemented method of training a machine learning model to predict output information related to an epidemic outbreak in a geographic region, comprising:
- obtaining temporal and geospatial data relative to said geographical area over a series of successive time periods from a plurality of data sources, comprising satellite imagery;
- forming a training feature table comprising a plurality of feature vectors, respectively associated with a plurality of cells of a cell grid mapped across the geographical area and over the series of successive time periods, one of said feature vectors comprising features extracted from said temporal and geospatial data and being associated with label data indicating at least if an epidemic outbreak has occurred or not during a time period of the cell; and
- training the at least one machine learning model based on the training feature table.

FIG. 2

EP 4 765 159 A1

**Description**

TECHNICAL FIELD

The present disclosure relates to a computer implemented method and system of training a machine learning model to predict an epidemic outbreak in a geographical area.

BACKGROUND

Zoonotic diseases are those which can pass from one species to another. They are ancient; genetic evidence demonstrates their presence in early humans as far back as 10,000 years ago. Well-known examples include SARS, MERS, Ebola and COVID-19. These diseases may be viral, bacterial, microbiological, or fungal, and different types can affect various species across different taxonomic ranks. Each type of zoonotic disease may have distinct pathways and mechanisms for transmission, impacting a wide range of animals and humans in diverse ways.

Over 60% of all emerging human infectious diseases worldwide can be attributed to zoonotic origins. As the frequency and prevalence of zoonotic diseases increase worldwide, investigating how wild animals and other environmental factors determine patterns of human disease, as well as predicting which regions are at greatest risk for future zoonotic disease emergence, is extremely important for public health.

There are a number of ecological, environmental, and human factors that are known to affect zoonotic disease risk. Habitat destruction, such as deforestation leading to urbanisation or agriculture expansion, forces wildlife into closer contact, thus facilitating the transmission of pathogens. Climate change also plays a significant role by altering ecosystems and enabling the spread of disease vectors, such as mosquitoes, into new areas.

Thus, there are existing surveillance systems which can be used for detecting and controlling zoonotic diseases in their emergent stages. These include deforestation monitoring systems; specific ecological, environmental, and economic databases; and networks of public health agencies and research institutions reporting in-field.

However, they are unable to systematically take into account all the environmental and contextual factors involved in the transmission and spread of diseases and, in particular to catch how the many zoonotic disease risk factors interplay. Therefore, there is still a need for an effective integrated framework for automatic zoonotic disease prediction, that encapsulates the intricacies of zoonotic dynamics.

The present invention improves the situation.

SUMMARY

According to a first aspect, the present disclosure concerns a computer-implemented method of training a machine learning model to predict an epidemic outbreak in a geographic region, comprising the steps of:

- obtaining temporal and geospatial data related to said geographical area over a series of successive time periods from a plurality of data sources, comprising satellite imagery, said temporal and geospatial data belonging to a group comprising natural and infrastructure data, demographic data, climate data, historic disease outbreak data, and vegetation condition data;

- building a training feature table, said training feature table comprising a plurality of feature vectors respectively associated with a plurality of cells of a cell grid mapped across the geographical area and over the series of successive time periods, said vector comprising features extracted from said temporal and geospatial data and being further associated with label data indicating at least if an epidemic outbreak has occurred or not during a time period of the cell;

- training the at least one machine learning model to predict output information related to an epidemic outbreak in the geographical area for a next time period from vectors of the training feature table associated with a current time period.

Thanks to the invention, a machine learning model is trained to predict epidemic outbreaks in cells of a geographical area from geospatial data related to this geographical data and coming from a wide range of data sources comprising relevant data relative to ecological, environmental and human factors known to be drivers of a plurality of diseases. The proposed method applies to any type of disease, in particular to zoonotic diseases.

The label data may of various types, depending on what the final user is interested in. In an embodiment, the label data comprises two classes, which are a disease class and a ['None'] disease class. In another embodiment, the label data comprises a disease type class label indicating a type of disease among a predetermined plurality of disease classes, comprising the ['None'] disease class. In an embodiment related to zoonotic diseases, the classes may be grouped by affected taxonomic class, for instance a bird disease class, a mammal disease class, etc, and a ['None'] disease class.

According to another example, the diseases may be grouped by viral family.

Then, the output information may thus take various forms depending on the used label data. In an embodiment, it comprises the disease class associated with the highest predicted estimate of probability or confidence score. The results may be presented to the user in any user friendly from, as a graphical representation, for instance a color or heat map.

In an embodiment, the method further comprises the step of preprocessing the temporal and geospatial data comprising forming the cell grid across the geographical area and over the series of successive time periods and extracting the data features from the temporal and geospatial data intersecting the plurality of cells. This allows to direct form one feature vector per cell.

In an embodiment, the preprocessing step comprises converting the temporal and geospatial data into a common coordinate system before extracting the data features from the converted temporal and geospatial data intersecting the plurality of cells. This allows to work with comparable temporal and geospatial data.

In an embodiment, the preprocessing further comprises filtering the label data by suppressing duplicates before forming the vectors of the training feature table and labelling the cells. This is to avoid introducing significant class imbalance within the training dataset.

In an embodiment, the geospatial data comprises locations of natural and infrastructure objects of interest in the geographical area and extracting data features comprises calculating a minimal spatial distance between the cell and nearby objects of interest and storing the calculated minimal spatial distances in the vector of the cell. The points of interest were chosen with the help of experts in (animal) epidemiology, because they have impact on zoonotic disease transmission. Intuitively, if the cell is for example next to a seaport, it is likely animals are being (illegally) transported through the cell, increasing the likelihood of zoonotic disease transmission. Also, certain types of animals may only live near lakes, which could make it more likely for some types of zoonotic disease to be present and less likely for others.

In an embodiment, the temporal and geospatial data comprises animal disease outbreak reports in the geographical area and extracting data features comprises calculating an inverse distance metrics between the cell and nearby cells where a previous epidemic outbreak was observed to have occurred within a given time range and storing the inverse distance metrics in the vector. If an outbreak has been recently reported close to a given grid cell, it is more likely that this disease has spread into the surrounding area. We use an inverse distance because the closer this outbreak was seen (in both space and time), the more likely it will spread.

In an embodiment, temporal and geospatial data comprises bushmeat activity reports in the geographical area and extracting data features from the other context related data comprise calculating an inverse distance metric between the cell and nearby cells where bushmeat activities were observed to have occurred within a given time range and storing the inverse distance metric in the feature vector.

In an embodiment, the temporal and geospatial data comprises locations of vegetation condition alerts associated with a date of disturbance and a confidence level and extracting data features comprises calculating determining a number of high-confidence vegetation condition alerts having occurred within the cell and storing the number of high-confidence vegetation condition alerts in the feature vector. Vegetation condition has an influence on wildlife habitats (who would we expect to see in an area), is an indicator for human-wildlife interaction, and can support different ecological dynamics. For instance, vegetation condition alerts are deforestation alerts. For instance, intact forests support diverse wildlife whereas sparse ones (through deforestation) can support rodents as predators may disappear, and mosquitos (prevalent zoonotic disease carriers) tend to thrive.

In an embodiment, the label data comprises a disease class among a plurality of disease classes comprising a ['None'] disease class, the building step comprises selecting feature vectors having a label class of disease equal to the ['None'] disease class, or ['None'] labelled feature vectors, clustering the ['None'] labelled feature vectors into a number K of clusters based on a minimum spatial distance criteria, K being an integer smaller than a number of ['None'] labelled feature vectors in said training feature table, and filtering said training feature table by retaining a same number N of ['None'] labelled feature vectors per cluster.

This is because most grid cells have the disease class label "[none]". This under sampling allows selecting a small proportion of ['None'] labelled vectors and thus avoiding to get a biased machine learning model. In an embodiment, clustering is performed on a month-by-month basis across multiple years, which allows extracting seasonal trends. In an embodiment, a same number of ['None'] labelled feature vectors per cluster is selected per time period over the series of time periods.

In an embodiment, the method further comprises the steps of determining measures quantifying a contribution of the features of the training feature table to the output information predicted by the machine learning model and providing the measures to a user. An advantage is to allow for interpretable explanations of how features influence outcomes. It may help the user updating the training feature table and improving the accuracy of the output prediction.

In an embodiment, Shapley values are calculated. Shapley values are model-agnostic. Unlike techniques like partial dependence plots (PDPs), which may require specific model structures, They ensure a fair and mathematically rigorous allocation of credit to each feature by considering all possible subsets of features and they are particularly well suited for complex models requiring trustworthy, interpretable explanations. In an embodiment, tree-path-dependent Shapley values are estimated based on a tree-based model built to input the same feature vectors as the MLM and to output the most probable disease. In this way, the structure of decision trees is exploited to quickly calculate estimates.

In an embodiment, the method further comprises the steps of detecting a new disease observed to have occurred in the geographical area from one or more of the plurality of data sources, updating the label data and building an updated training feature table comprising feature vector labelled with the new label data; and retraining the at least one machine learning model based on the updated training feature table. This allows the model to evolve so that it remains effective and efficient over time, with the ability to take into account the emergence of new viruses.

According to a second aspect, the present disclosure relates to a computer implemented method of predicting an epidemic outbreak in a geographical area, including the following steps, performed by a computer system:

- obtaining temporal and geospatial data relative to said geographical area for a current time period from a plurality of data sources, comprising satellite imagery, said temporal and geospatial data belonging to a group comprising natural and infrastructure data, demographic data, climate data, historic disease outbreak data, and vegetation condition data;

- building an input feature table, said input feature table comprising a plurality of feature vectors, respectively associated with a plurality of cells of a cell grid mapped across the geographical area for the current time period, one of said feature vectors comprising features extracted from said temporal and geospatial data intersecting with the associated cell during the current time period of the associated cell; and

- predicting, based on the input feature table and by using at least one trained machine learning model, output information indicating at least if an epidemic outbreak is likely to occur or not in the cell during a next time period;

- providing prediction results based on the output information to a user through a human-machine interface of the computer system.

Thanks to the invention, an estimate of probability of an epidemic outbreak in a geographical area in a short term is automatically predicted based on various types of geospatial data that are known to convey potential drivers of disease risks. For instance, prediction is provided for next month based on geospatial data collected during a current month. With the invention, a user is informed on request or automatically on a regular basis or depending on a predicted level of risk.

In an embodiment, the output information may comprise an estimate of probability of a disease outbreak for one or more disease classes from a plurality of diseases classes that were observed to have occurred in the geographical area. In another embodiment, the output information comprises the highest probability score and the associated class of disease.

In an embodiment, providing prediction results to a user comprises deriving a risk indicator from the probability score, said risk indicator belonging to a group comprising low risk when the probability score is less than 90%, medium risk when the probability score is between than 90% and 95%, high risk when the probability score is between 95% and 99% and very high risk when the probability score is above 99%.

In an embodiment, the method comprises the step of preprocessing the temporal and geospatial data comprising forming the cell grid across the geographical area and over the series of successive time periods and extracting the data features from the temporal and geospatial data intersecting the plurality of cells.

In an embodiment, the predicting step is carried out by a first and a second trained machine learning models, based on the feature table, the first trained machine learning model being configured to output, for one cell of the cell grid, an estimate of a highest probable class of disease among a plurality of classes of diseases, and the second trained machine learning model being configured to output a taxonomic class of animal predicted to be affected by the class of disease.

According to a third aspect, a computer system of predicting an epidemic outbreak in a geographical area comprises:

- a communication component configured to obtain temporal and geospatial data relative to said geographical area for a current time period from a plurality of data sources, comprising satellite imagery, said temporal and geospatial data belonging to a group comprising nature and infrastructure data, demographic data, climate data, historic animal disease outbreak data, and vegetation condition data;

- a building component configured to build an input feature table, said input feature table comprising a plurality of vectors respectively associated with a plurality of cells of a cell grid mapped across the geographical area for the current time period, one of said feature vectors comprising features extracted from said temporal and geospatial data intersecting with the associated cell during the current time period of the associated cell;

- a predicting component configured to predict, based on the input feature table and by using at least one trained machine learning model, output information indicating at least if an epidemic outbreak is likely to occur or not in the cell during a next time period; and

- a human-machine interface configured to provide prediction results based on the output information to a user.

In an embodiment, the system comprises a training component configured to train the machine learning model to predict output information related to an epidemic outbreak in a geographic region, comprising:

- obtaining temporal and geospatial data related to said geographical area over a series of successive time periods from a plurality of data sources, comprising satellite imagery, said temporal and geospatial data belonging to a group comprising nature and infrastructure data, demographic data, climate data, historic disease outbreak data, and vegetation condition data;

- building a training feature table, said training feature table comprising a plurality of feature vectors, respectively associated with a plurality of cells of a cell grid mapped across the geographical area and over the series of successive time periods, one of said feature vectors comprising features extracted from said temporal and geospatial data intersecting with the associated cell and label data indicating at least if an epidemic outbreak has occurred or not during a time period of the associated cell;

- training the at least one machine learning model to predict output information related to an epidemic outbreak in the geographical area for a next time period from feature vectors of the training feature table associated with a current time period.

In an embodiment, the system comprises at least one processor and at least one memory including computer program code, the at least one memory and computer program code configured to, with the at least one processor, cause the system to carry out the steps of the method according to the first and the second aspects.

According to a fourth aspect, a computer program comprises instructions that, when executed by a processor, cause the processor to perform the method(s) according to the first and/or the second aspects. The instructions may cause the processor to perform one or more or all steps of a method according to the first aspect.

Correlatively, a non-transitory computer-readable medium comprises program instructions stored thereon for causing a computer to perform the method according to the first and/or the second aspects. The non-transitory computer-readable medium, the computer program and the system may present the same advantages as the method(s) according to the first aspect and/or the second aspects.

BRIEF DESCRIPTION OF THE DRAWINGS

Other features, purposes and advantages of the disclosure will become more explicit by means of reading the detailed statement of the non-restrictive embodiments made with reference to the accompanying drawings.

Figure 1 shows, according to an embodiment an architecture of a system of predicting epidemic outbreaks in a geographical area.

Figure 2 is a flowchart of a method of training a machine learning model to predict epidemic outbreaks in a geographical area, according to an embodiment.

Figure 3 details steps of preprocessing geospatial and temporal data and of building a training or an input feature table to be applied to the machine learning model, according to an embodiment.

Figures 4A to 4D show exemplary grid maps of a geographical area captured by satellite imagery.

Figures 5A and 5B respectively show percentages of the different disease classes in the feature

table respectively when the label data have been filtered out or not to remove duplicates.

Figures 6A and 6B schematically show exemplary structures of a machine learning model used by the system of predicting epidemic outbreaks, according to an embodiment.

Figure 7 schematically illustrates a cross-fold validation process for training a machine learning model, in an embodiment.

Figures 8A and 8B show exemplary confusion matrices obtained from the predictions of the trained machine learning model, with two different groups of disease classes.

Figure 9 shows temporal and spatial distances from false positive points to nearest positive neighbor, in an embodiment.

Figure 10 shows, according to an embodiment, an exemplary graphical representation of measures quantifying respective contributions of the features of the vectors of the input feature table to the output information predicted by trained the machine learning model.

Figure 11 is a flowchart of a method of predicting epidemic outbreaks in a geographical area, using a trained machine learning model, according to an embodiment.

Figures 12A and 12B show exemplary graphical representations of prediction results provided to a user according to an embodiment.

Figure 13 schematically presents a hardware structure of a system of predicting epidemic outbreaks in a geographical area, according to an embodiment.

DETAILED DESCRIPTION

The specific structural and functional details described herein are non-limiting examples. The example embodiments described herein are subject to various modifications and alternative forms. The subject matter of the disclosure may be embodied in many different forms and shall not be construed as being limited to the only embodiments presented herein as illustrative examples. It should be understood that there is no intention to limit the embodiments to the particular forms described in the remainder of this document.

In this disclosure, a method and system are proposed to automatically predict an epidemic outbreak in a geographical area based on geospatial and temporal data from various data sources and using a trained machine learning model. Correlatively, a method and system are proposed to train a machine learning model to predict epidemic outbreaks in a geographical area based on geospatial and temporal data from various data sources.

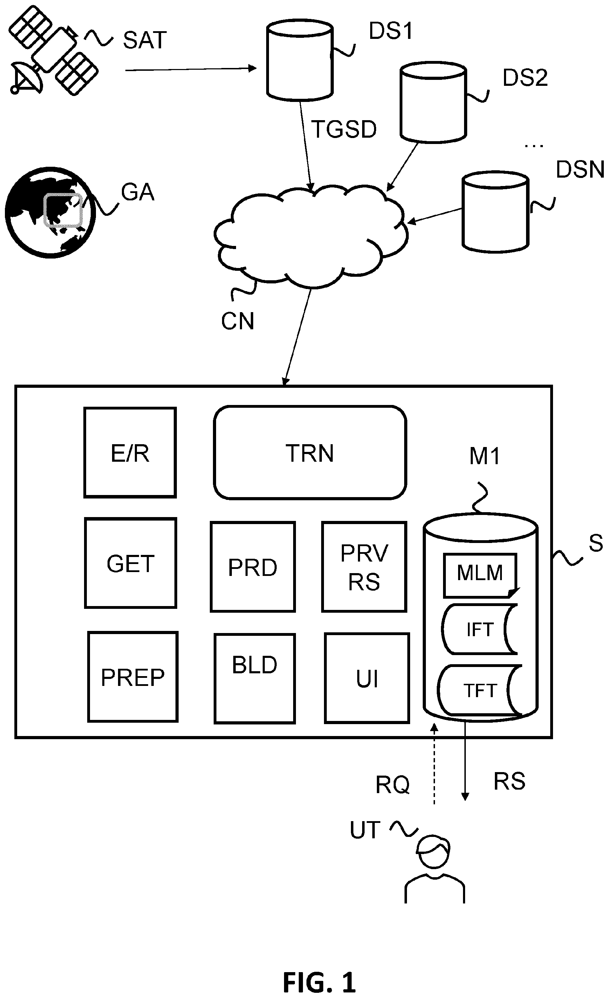

Figure 1 shows, according to an embodiment, a functional architecture of a computer system S predicting an epidemic outbreak in a geographical area, comprising a communication component GET configured to obtain temporal and geospatial data relative to a geographical area GA of planet earth from a plurality of data sources DS1-DSN, with N an integer greater or equal to 1. They may be received by an emission/ reception component E/R of the computer system S through a telecommunication network CN. This temporal and geospatial data TGSD may comprise or be derived from raw image data captured by satellite imagery SAT during a current time period, for example the current month, and belongs to a group comprising economic and infrastructure data, climate data, medical facilities data, human and species census data, wildlife and conservation data and topography and elevation data, more generally any type of contextual data relevant to disease proliferation. The temporal and geospatial data is stored in a memory or storage device M1 of the computer device S.

In the embodiment of Figure 1, the computer device S further comprises a building component BLD configured to build an input feature table IFT comprising a plurality of feature vectors. The feature vectors are each associated with a corresponding cell of a plurality of cells of a cell grid mapped across the geographical area GA and with the current time period. A feature vector comprises a plurality of features, extracted from the temporal and geospatial data intersecting the associated spatiotemporal grid cell.

In the example of Figure 1, the computer system S further comprises a predicting component PRD configured to predict, based on the input feature table IFT and by using at least one trained machine learning model MLM, stored in a memory for example the memory M1 of the computer system S, output information EOI related to an epidemic outbreak in the geographical area for a next time period from vectors of the training feature table associated with a current time period.

The computer system S further comprises a human-machine interface UI and a provision component PRV configured to provide prediction results RS based on the output information EOI to a user US through the human-machine interface UI. The prediction results may be provided to the user UT in a user-friendly manner, for example as a visual and graphical representation.

According to an embodiment, the computer system S further comprises a preprocessing component PREP configured to convert the temporal and geospatial data into a common coordinate system, forming the cell grid across the geographical area GA and on the current time period and extract the data features from the converted temporal and geospatial data intersecting the plurality of cells of the cell grid.

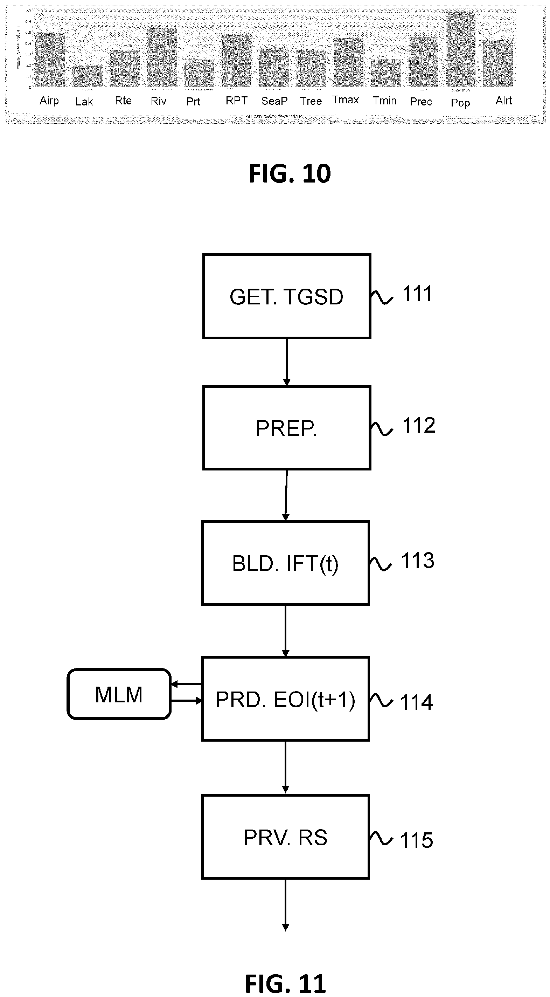

In an embodiment, the computer system S is configured to implement a method of predicting an epidemic outbreak in a geographical region that will be further described in relation to Figure 11.

In the example of Figure 1, the computer system S further comprises a training component TRN configured to train a machine learning model MLM to predict output information related to an epidemic outbreak in a geographic region. In an embodiment, the training component TRN is configured to get temporal and geospatial data, relative to the geographical area over a series of successive time periods from a plurality of data sources, for example the data sources DS 1-DSN and to build a training feature table TFT comprising a plurality of feature vectors associated each with one cell of a plurality of cells of a cell grid mapped across the geographical area and over the series of successive time periods. The training feature table further comprises label data associated with each of the feature vectors comprising at least a class of disease observed within the cell during the associated time period, among a plurality of disease classes. The training feature table TFT is stored in a memory, for example the memory M1.

The training component TRN is further configured to train the machine learning model MLM based on the training feature table TFT.

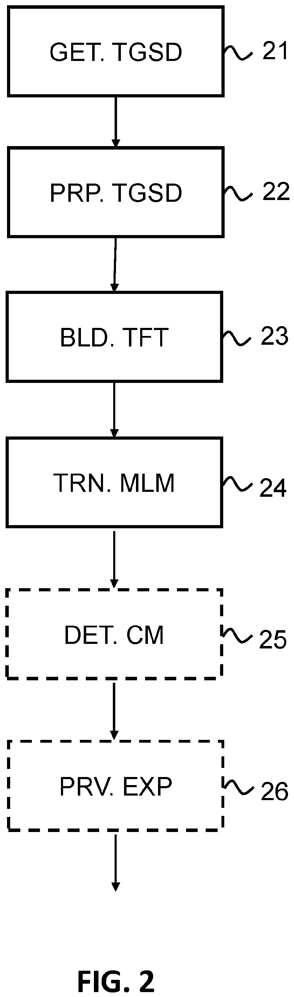

In an embodiment, the computer system S is configured to implement a method of training a machine learning model to predict an epidemic outbreak in a geographical region that will be further described in relation to Figure 2.

In another embodiment, the method of training a machine learning model is implemented by another computer system (not represented in Figure 1), distinct from the computer system S. In this case, once trained, the MLM is provided to the computer system S. For instance, the trained MLM is stored in a remote memory or storage device that is accessible to the computer system S or transmitted to the computer.

In an embodiment, the system S is configured to cause the training component TRN to retrain the ML model. Optionally, this retraining may be triggered on a regular time basis, depending on an update frequency of the data sources, for example twice a year or upon detection of a new disease observed to have occurred in the geographical area. If the model works well, there is no need to train it very frequently. For instance, if a type of temporal and geospatial data is updated 100 times a year, it would not be necessary to train the model each time the data is updated, if the performance gap is not relevant. So, the model could be trained once the data has been updated 30 times such that the training dataset would be sensibly modified, which could provide a positive performance gap.

The training component is configured to update the plurality of disease classes by integrating the new class disease and by building an updated training feature table comprising feature vector labelled with the new disease class; and to retrain the machine learning model based on the updated training feature table.

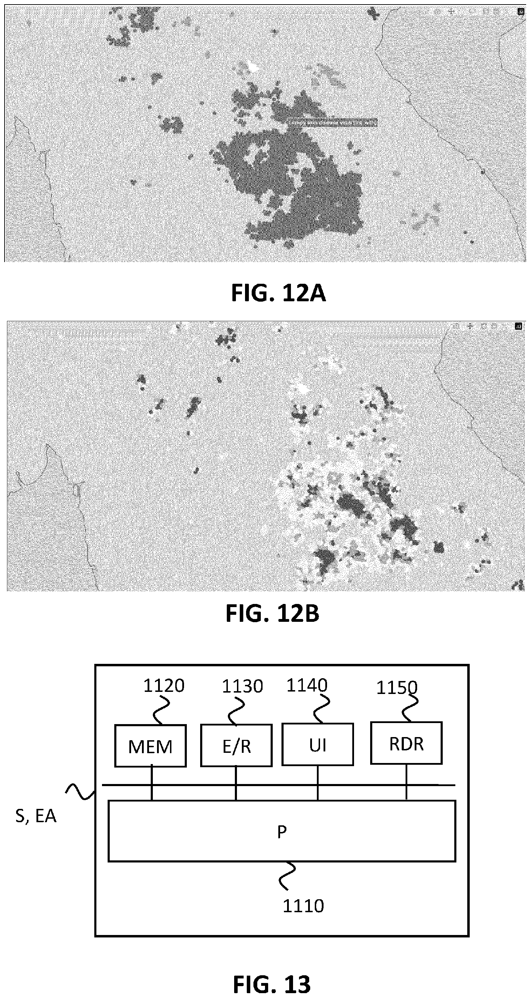

In an embodiment, the computer system S is an electronic apparatus EA with the hardware structure of a computer and comprises one or more operably connected to one or more memory or storage devices, as will be described hereinafter in relation to Figure 13. In another embodiment (not represented), the computer system S is implemented in a distributed manner, for example as a cloud platform within a cloud computing architecture. The user UT can access the prediction results on request by connecting to a web page of the system S from their terminal or receive them regularly via communication channels.

In the following, we will consider as an illustrative example of a geographical area GA, the Greater Mekong Subregion (GMS). This a transnational area in Southeast Asia encompassing Cambodia, China (Yunnan and Guangxi provinces), Laos, Myanmar, Thailand, and Vietnam. It is characterized by rich biodiversity, extensive river systems (including the Mekong River), and diverse ecosystems. The region is known for its rapid economic development, agriculture, tourism, and energy resources, while facing challenges such as environmental degradation and climate change. Its rich biodiversity, dense human and animal populations, and extensive human-wildlife interaction make GMS highly vulnerable to zoonotic diseases. In particular, factors such as deforestation, agricultural expansion. Traditional practices such as wet markets also contribute to the transmission of diseases.

The GMS region has experienced outbreaks of zoonotic diseases among which the most prevalent ones recently are Lumpy Skin Disease Virus (LSD), High pathogenicity Avian Influenza (HPAI), and African Swine Fever Virus (ASF). Surveillance and healthcare infrastructure are often inadequate, particularly in rural areas. While some efforts are made to adopt a unified approach integrating human, animal, and environmental health, coordination across borders remains a challenge. Enhanced surveillance, cross-sector collaboration, and sustainable environmental practices are critical to mitigating these risks.

A method of training a machine learning model to predict an epidemic outbreak in a geographical area will now be described in relation to Figure 2, according to an embodiment. It may be implemented by the computer system S of Figure 1 or by any other implementation of said system.

In a step 21, temporal and geospatial data TGSD related to a geographical area GA over a series of successive time periods are collected from the plurality of data sources DS 1-DSN.

They comprise comprising raw image data captured by satellite imagery SAT and other context related data belonging to a group comprising economic and infrastructure data, demographic data, climate data, historic disease outbreak data, and vegetation condition data.

More generally, the collected data need to be of varied data types to provide valuable information to the machine learning model. As illustrative example, we cite:

- Economic and Infrastructure Data: This data covers transportation networks, energy facilities, and urban development, which are crucial for understanding economic and infrastructure attributes.
- Medical Facilities: Data on medical facilities helps assess healthcare accessibility and readiness and may highlight reporting biases.
- Human and Species Census Data: Demographic information on both humans and wildlife is essential for understanding population distribution, density, and potential interactions.
- Wildlife and Conservation Data: this data focuses on biodiversity, conservation efforts, and endangered species, emphasising environmental preservation.
- Satellite Imaging: Satellite imagery offers dynamic views of the geographical area over time, allowing for the observation of land use, vegetation, and hydrological changes. It also provides Topography and Elevation Data, allowing understanding the topographical layout and elevation variations is crucial for building an accurate representation of the area. This data aids in predicting population densities and species distributions. For example, areas separated by steep cliffs might have distinct populations despite being geographically close, etc.

In an embodiment, geospatial data may be obtained in various formats, each shedding light on different characteristics of the GA. These formats may include:

- Vector data (for example Shapefiles, GeoJSON): encodes geographic elements as points, lines, and polygons. This is well suited to capturing discrete features varying geometries, such as landmarks and infrastructure of the geographical area. Each geometry within the file has associated attributes for analysis.
- Raster images (for example GeoTiff): represent geographic data as a grid of pixels, each containing one or multiple values. It allows for storing continuous data such as satellite imagery, elevation models, and land cover maps.
- Tabular data with location details (for example CSV (Comma-Separated Values)): Data are stored in a structured, table-like format. This format is most useful for storing objects with numerous attributes, such as disease data. Each row encodes location data (latitude, longitude) alongside associated characteristics for that location (for example disease observed, animal observed).

While some of the datasets are purely geospatial, many also contain time-related data. This time dimension can be encoded in the following ways:

- As separate files: the dataset is comprised of multiple GeoTiffs / GeoJSONs, each corresponding to a different time period.
- Within the dataset: the time dimension is encoded within the dataset itself. This could be as a 'date' column within a tabular dataset, or a custom pixel encoded in image data.

Relevant temporal and geospatial data can be obtained from a multitude of open data sources. Illustrative examples of these open data repositories include:

- A data source comprising GMS physical and economic attributes, such as the GreaterMekong datasource, available at https://www.greatermekong.org/map-type/map-archive, comprising locations of various objects of interest, such as lakes, infrastructures and protected areas, in SHAPE files.
- A data source related to global forest watch information, such as the globalforestwatch data source comprising monitored real-time forest disturbance alerts from satellites, available at website https://www.data.globalforestwatch.org, as GeoTiff files. Time-related information is included.
- A data source related to global forest tree cover information, such as the Global Forest Change data source of the University of Maryland's Global Land Analysis and Discovery (GLAD) laboratory, available at https://www.storage.googleapis.com/earthenginepartners-hansen/GFC-2021-v1.9/download.html. This is a comprehensive dataset detailing global forest cover changes from 2000 to 2021. This dataset, derived from Landsat imagery, includes GeoTIFF files characterizing forest extent, loss, and gain over the specified period.
- A data source comprising population density information, such as WorldPop, available at website https://www.worldpop.org, which provides high-resolution datasets for individual countries from 2000 to 2020. These datasets are adjusted to match UN population estimates and are available in GeoTIFF format.
- A WAHIS (World Animal Health Information System) data source, which is an online platform developed by the World Organisation for Animal Health (WOAH), available at whoa.org, that is dedicated to collecting, verifying, and disseminating information on animal diseases, including zoonotic diseases that can affect humans. It comprises reports containing information on historic animal disease outbreaks, available as CSV files. They include time related information, and

- A climate data source such as WorldClim, available at the website https://worldclim.org, comprising monthly recorded temperature and precipitation data, available as Geo Tiff files including time related information.

In an embodiment, the collected temporal and geospatial data TGSD covers a range of successive time periods, typically several years, for instance from 2018 to 2022, inclusive of all months. The successive time periods are months. They are expressed in Year, Month temporal unit.

The collected temporal and geospatial data TGSD are stored in a memory, for example the memory M1 of the computer system S. In case of a distributed implementation, once source, the raw data TGSD is stored remotely in one or more object storage devices, designed to store unstructured data as objects, such as Google cloud® storage buckets.

It should be noted that most of the collected temporal and geospatial data relates to a wider region of the world than the geographical area of interest. Therefore, high resolution data geospatial data TGSD may be cropped before storage to only encompass the geographical area, which allows to streamline storage and computational demands. For instance, data related to deforestation alerts are cropped, whereas temperature data does not need to be cropped.

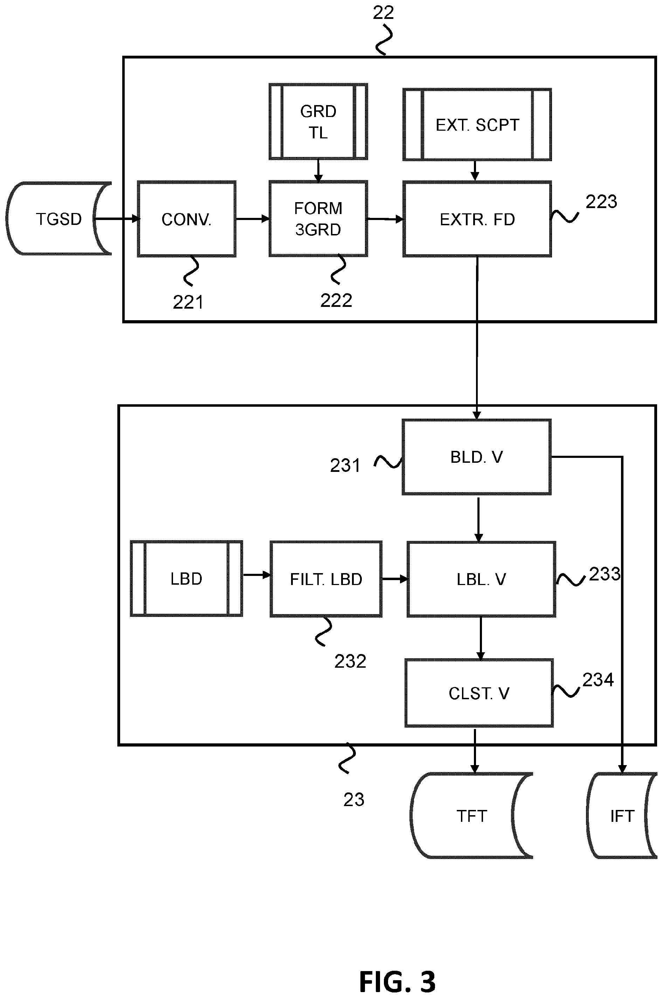

In a step 22, the temporal and geospatial data TGSD are preprocessed. This is required to extract meaningful attributes for the targeted prediction task. This step will be described in more details, according to an embodiment, in relation to Figure 3.

In a step 23, a training feature table a TFT is built from the pre-processed temporal and geospatial data. The training feature table TFT comprises a plurality of feature vectors, each feature vector being associated with one cell of a cell grid mapped across the geographical area and over the series of successive time periods, said vector comprising features extracted from said geospatial data and label data indicating at least if an epidemic outbreak has occurred or not during a time period of the cell. This step will also be further described in more details, according to an embodiment, in relation to Figure 3.

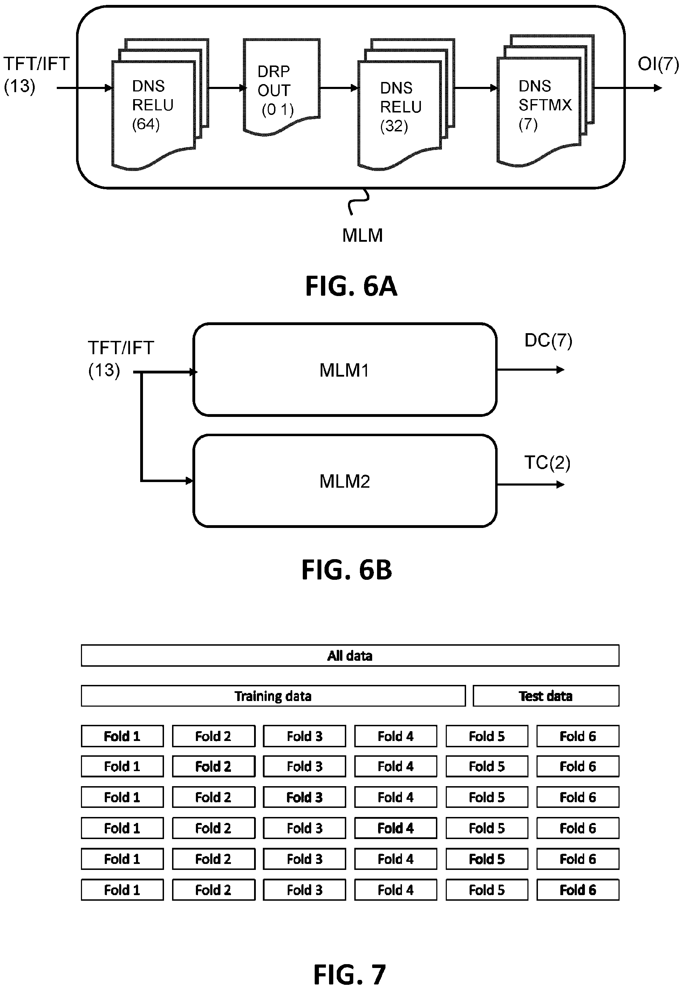

In a step 24, the training feature table is used to train a machine learning model MLM to predict output information related to an epidemic outbreak in the geographical area for a next time period from input vectors associated with a current time period. For example, the current and next time periods are months. In an embodiment, the machine learning model is trained to predict output information for each cell of the grid cell covering the geographical area. More details about a structure of the MLM will be described hereinafter in relation to Figure 6, according to an embodiment.

Referring to Figure 3, an embodiment of the pre-processing step 22 will now be described in more details.

In a sub-step 221, the geospatial data from TGSD are converted into a common coordinate system. In an embodiment, they are mapped to the common spatial reference system EPSG:4326, defined by the European Petroleum Survey Group (EPSG), and also known as WSG84 (World Geodetic System 1984)). EPSG 4326 is commonly used in global mapping, GPS systems, web mapping applications (like Google Maps® and OpenStreetMap®). It uses a geographic coordinate system, meaning coordinates are specified in terms of latitude and longitude and covers the entire globe. Units are degrees, axis orders are Latitude (Y) and Longitude (X). Based on the WGS 84 datum, the shape of the Earth is defined as a reference ellipsoid. This conversion operation allows to ensure cconsistency within the geospatial data and perform operations native to this system (for instance, using haversine distance as will be described hereinafter). Regarding temporal data, in case it was recorded every hour / day, it would be averaged out over the month.

In a sub-step 222, a cell grid SPT-GRD is formed across the geographical area GA and over the series of successive time periods. In an embodiment, a hexagonal grid is used. This enables to produce a tessellation across the geographical area GA. In an embodiment, use is made of a software tool GRD-TL, for example the Uber's open source h3 library. This is a geospatial indexing system that divides the Earth's surface into a hierarchical grid of hexagonal cells for efficient spatial analysis and data aggregation. The hierarchical grid supports multiple resolutions, with finer hexagons nested within larger ones, enabling analyses at varying levels of detail, which gives flexibility in defining the resolution of the cell grid.

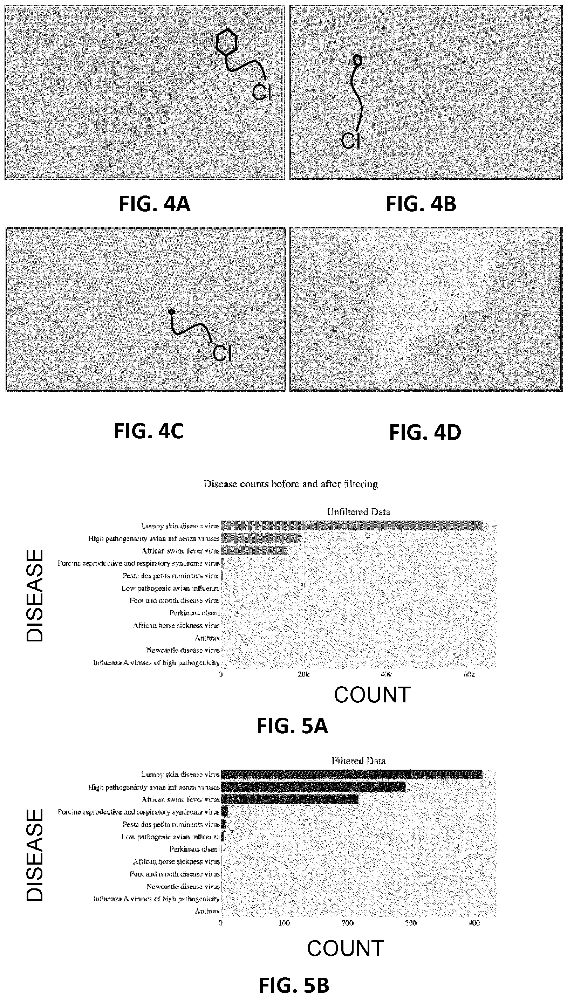

Referring to Figures 4A-4D, hexagonal grids are mapped across the GMS with four different resolution levels from the coarser to the finer chosen within a wider range of 1-15 resolution levels available in Uber h3. Figure4 corresponds to a coarse resolution level 4, Figure 4B to resolution level 5, Figure 4C to resolution level 6 and Figure 4D to resolution level 7. In Figures 4A-4C an exemplary cell CI is shown. The cells are not visible in Figure 4D, because they are too small.

Table 1, taken from Uber's documentation (https://h3geo.org/docs/core-library/restable/), presents cell statistics across resolution levels 4-7 shown in Figures 4A-4D.

**Table 1**

| Resolution | Average hexagon area (km$^2$) | Average edge length (km) | Number of cells |
|---|---|---|---|
| 4 | 1,770.347654491 | 26.07175968 | 1,165 |

(continued)

| Resolution | Average hexagon area (km$^2$) | Average edge length (km) | Number of cells |
|---|---|---|---|
| 5 | 252.903858182 | 9.854090990 | 8,190 |
| 6 | 36.129062164 | 3.724532667 | 57,313 |
| 7 | 5.161293360 | 1.406475763 | 401,095 |

It is noteworthy that areas / edge lengths are computed with a spherical model of the Earth using the authalic radius given in the EPSG:4326 (WGS84) coordinate system. In an embodiment, a resolution of six is chosen for the grid cell, it strikes a balance between being detailed enough to capture small-scale geographic differences while minimizing computation time. With resolution level 6, the cell grid spanning the GMS has around 57,000 cells.

To capture changes over time within the geographic area, the cell grid is extended to the temporal dimension by assigning each cell a specific time period, for instance in month and year. Within this scheme, each cell within the grid now represents a snapshot in time of a given location, effectively creating a three-dimensional grid or spatiotemporal grid 3D-GD where the added dimension is time.

Back to Figure 3, in a sub-step 223, data features are extracted from the converted temporal and geospatial data for each cell of the spatiotemporal cell grid SPT-GRD. In an embodiment, the following operations are performed, for each grid cell and the temporal and geospatial data:

Spatially intersect the grid cell with the geospatial data or dataset. If applicable, the temporal part of the grid cell is considered by filtering out values which are not associated with the time period, for example year and month, of the given grid cell.

Aggregate the temporal and geospatial data intersecting with the grid cell in a way appropriate for each data source so as to extract relevant features that will allow the machine learning model to predict epidemic outbreaks.

In an embodiment of sub-step 223, the other context related data comprise locations of objects of interest in the geographical area GA. These locations may originate from the aforementioned GreaterMekong datasource. The objects of interest may be natural, such as lakes or rivers. They may also correspond to human made infrastructures, such as airports, seaports, trade routes, RPT infrastructure nodes, and protected areas. A Regional Power Trade node corresponds to infrastructure ensuring the physical flow of electricity across regions. The data source may contain line-like data of transmission lines important to RPT and Interconnections. This is an important feature as such a node can only exist with some agricultural disruption to build power lines through natural ecosystems.

For each grid cell, the locations of objects of interest are used along with the spatial coordinates of the grid cell to calculate a minimal spatial distance between the grid cell and nearby cells comprising the objects of interest. The extracted features are the calculated minimal spatial distances. As this data source is static, the time dimension of the cell does not come into play.

In another embodiment, the other context related data comprise population density data. They may originate from the aforementioned WorldPop data source. For example, data is organised into separate density image files by year. Thus, the year dimension of the 3d grid is used to select the relevant file. From there, each grid cell is spatially intersected with the density image and a mean population density value across the land-based pixels is calculated. Land-based pixels are meant to design pixels that are in the land, not over water (river, lake or sea, for example (e.g. South China Sea, Mekong River for the GMS), that is pixels where population may live. Water pixels are filtered out as it does not make sense to calculate population density there. In this case, the extracted feature is thus a mean population density value per grid cell.

In another embodiment, the other context related data comprise temperature and precipitation data. They may originate from the aforementioned Worldclim data source. Here, the data is organised into separate image files by both year and month. Thus, it may be proceeded in a similar way as in the population density case to filter out the temperature and precipitation image files, this time considering month as well as year. Each grid cell is spatially intersected with the temperature and precipitation image and a mean temperature value and a mean precipitation value across the land-based pixels is calculated. In this case, the extracted feature is thus a mean temperature value and a mean precipitation value per grid cell.

In yet another embodiment, the other context related data comprise integrated deforestation alerts. They may originate from the aforementioned globalforestwatch data source. In the alert images provided by this data source, each pixel encodes a date of disturbance and an associated confidence level in one integer value.

The leading integer denotes confidence, and the following integers denote the number of days since 31st December 2014. The leading integer of the decimal representation is 2 for a low-confidence alert, 3 for a high-confidence alert, and 4 for an alert detected by multiple alert systems, followed by the number of days since December 31, 2014. 0 being the no-data value (no alert). For example: 20001 is a low confidence alert on January 1st, 2015, 30055 is a high confidence alert on February 24, 2015, 21847 is a low confidence alert on January 21, 2020, 41847 is a highest confidence alert (detected by multiple alert systems) on January 21, 2020. Alert date represents the earliest detection.

Each grid cell is spatially intersected with the alert image to capture all alerts that have occurred in the sub-area corresponding to the grid cell. Then, by matching the date of the grid cell with the decoded date of disturbance, we can determine the number of high-confidence alerts that have occurred within time period (year and month) of the grid cell. In this case, the extracted feature is thus the number of high-confidence forest disturbance alerts per grid cell.

In yet another embodiment, the other context related data comprise forest cover extent image data. They may originate from the aforementioned Global Forest Change data source. Each grid cell is spatially intersected with a forest cover extent image file whose pixels comprise a percentage value, of how much of the land is covered by forest canopy.

An average value of forest cover extent us is calculated over the pixels of the grid cell to get an average forest cover percentage for the grid cell. Similarly to the GMS data, this data source is time-independent, so the time component of the cell does not come into play. In this case, the extracted feature is thus an average value of forest cover extent per grid cell.

In yet another embodiment, the other context related data comprise information on historic animal disease outbreaks. In an embodiment, this is a per-report representation. Each row has a date of report, location of report, disease name. Nearby cells may be defined as ones within 3 hexagons away. This approximately corresponds to a latitude and longitude range, which is then used to search for other outbreaks in the table having occurred within this latitude, longitude range, within the last three months. They may originate from the aforementioned WAHIS data source or any other related one. For a given grid cell, we consider nearby cells which have had an outbreak occur within them within the past three months. Denote these outbreaks as $\{x_i\}_i$. We then define an inverse distance metric as:

$$D(cell) = \sum_i \sqrt{\frac{\alpha}{h(cell, x_i)^2} + \frac{\beta}{t(cell, x_i)^2}}, \quad \text{(Equation 1)}$$

where $h$ denotes the spatial haversine distance, which is a great-circle distance between two points on the surface of a sphere, calculated using their latitude and longitude coordinates. This metrics accounts for the Earth's curvature, providing a more accurate distance than planar approximations. $t$ denotes time difference in seconds, and $\alpha$, $\beta$ are weights to ensure the distances fall on the same scale. These distances calculated using the cell's centroid, in both space and time. In this case, the extracted feature is thus the calculated value of D per grid cell. Indeed, if an outbreak has been recently reported close to a given grid cell, it is more likely that this disease has spread into the surrounding area. Therefore, an inverse distance because the closer this outbreak was seen (in both space and time), the more likely it will spread.

Therefore, in an embodiment, the extracted features per grid cell comprise:

- a minimal spatial distance to objects of interest present in the grid cell, comprising RPT nodes, airports, seaports, trade routes, lakes, rivers and protected areas,
- a mean population density value,
- a mean temperature value,
- a mean precipitation value,
- a number of high-confidence forest disturbance alerts,
- an average value of forest cover extent,
- a value of distance D to recent disease outbreaks.

Of course, the disclosure is not limited to the above list of extracted features. In other embodiments, other relevant features may be extracted from other context related sources. For example, bathymetry and topology data (encompassing land type, elevation, aspect, and roughness) could be harnessed as it could have an impact on the likelihood of a disease to spread (e.g. animals may be more likely to travel downhill and spread disease).

According to another example, one or more features are extracted from information about bushmeat activities. Bushmeat or wild meat refers to the meat of terrestrial wild mammals hunted primarily for human consumption in tropical and subtropical regions. Illegal wildlife trade is known as an important driver of zoonotic diseases. Animals, or their parts, are often kept in unsanitary conditions which raises zoonotic disease risk, and their transport into new areas can introduce novel zoonotic diseases into ecosystems. A similar metric could be calculated as described above for zoonotic disease alerts, where number of illegal bushmeat trade activities that have occurred within a grid cell and time period are counted. The metric is then integrated to the feature vector and passed to the ML model. Alternatively, if a third party mathematical model were built to return a probability of bushmeat activities occurring, this probability could be integrated to the feature vector and directly applied to the MLM.

In an embodiment, this feature extraction is performed on a feature-by-feature basis, for all grid cells at once. This allows to minimise data read-in times, and to globally accelerate extraction.

Referring back to figure 3, an embodiment of the building step 23 will now be described in more details.

In a sub-step 231, a feature vector V per grid cell is formed with the aforementioned extracted features. It is associated with a vector ID, spatial coordinates and a date value, but these fields are not included into the feature vector itself, because if they were passed to the MLM, they would add bias.

In a sub-step 233, label data LBD is obtained for each feature vector and associated with it to form a pair. The label data comprises a disease class label from a plurality of diseases classes defined for the geographical area GA. In an embodiment, the label data is extracted from a data source reporting the disease outbreaks in the geographical area over the considered series of successive time periods. For example, the label data may originate from reports available in the aforementioned WAHIS data source, whose data are cropped for the Mekong basin GA.

To label the 3d grid cells with a disease class, a similar approach as described for feature extraction may be used. the cropped data from the data source so that it only contains disease records having occurred in the grid cell. From there, various types of labels may be generated.

A disease class label that may include a list of all disease types observed within the cell. If the filtered dataset returns no data, the disease class is put to ['None']. As the grid cells are small, the list often turns out to be reduced to a single disease class, but there also may be multiple occurrences of disease classed for a given cell. The proposed model training process is designed to be robust and account for this.

In an embodiment, the disease class label comprises a disease type class among a predetermined plurality of disease classes. For zoonotic diseases, the classes may be grouped by affected taxonomic class, for instance a bird disease class, a mammal disease class and a no disease class. According to another example, the diseases may be grouped by virus. For instance, for the GMS, the disease classes may belong to a group comprising: lumpy skin disease virus, high pathogenicity avian influenza virus, African swine fever virus, African horse sickness virus, porcine reproductive and respiratory syndrome virus, New castle disease virus, Anthrax, Foot and Mouth disease virus, "peste des petits ruminants" virus, Perkinsus Olseni virus, anthrax, etc. In alternative, they may be grouped by viral family. Regarding the aforementioned prevalent viruses in the GMS, the Lumpy skin disease virus belongs to a Poxviridae group, the African swine fever virus to a Asfarviridae group, the High pathogenicity avian influenza viruses / Low pathogenic avian influenza to an Orthomyxoviridae group, the Newcastle disease to a Paramyxoviridae groupn the African horse sickness virus to a Reoviridae group and the Porcine reproductive and respiratory syndrome virus to a Arteriviridae group.

In yet another embodiment, the label data further comprise an affected class label comprising a list of taxonomic classes, for instance a bird class, a mammal class, etc, that can be affected by diseases observed within the grid cell. Most of the time, the affected taxonomic class label only includes one class item. Then, the feature vectors of the training feature table TFT are associated with a disease class, for instance classes grouped by viral family, along with an affected taxonomic class.

In yet another embodiment, the label data only comprises an outbreak class label comprising to classes, an outbreak class and a no outbreak class. The outbreak class label may bet set to 1 if any disease is observed to have occurred within the cell and 0 otherwise.

It is worth noting that the the label data is extracted from one or more disease reports, for instance originating from the aforementioned WAHIS data source, and contains a lot of duplicates. As these duplicates would introduce significant class imbalance within the training dataset. a step 232, filters out the extracted label data LBD to suppress duplicates. This is to avoid introducing significant class imbalance within the training feature table TFT.

At the end of sub-step 233, all the feature vectors are associated with label data, forming a training dataset of pairs of feature vectors, label data (V, LBD). In an embodiment, a training feature table TFT is built whose rows are the (V, LBD) pairs.

This is illustrated by Figures 5A and 5B showing respectively the class distribution of the vector dataset without or with the removal of duplicates from the label data.

As shown by Figure 5B, despite filtering of the label data at sub-step 232, the training dataset is still imbalanced. This is because the vast majority of grid cells have the label ['None']. A consequence is that is a machine learning model were trained on the entire dataset, the model would be extremely biased.

Optionally, in a sub-step 234, a down sampling process is undergone by selecting in the training feature table TFT the feature vectors V having a ['None'] disease class label, clustering the selected feature vectors into a number K of clusters based on a minimum spatial distance criteria. In an embodiment, the clustering is done on a month-by-month basis. For each month, a set of spatially representative points is taken, across multiple years (e.g. Jan 2018, Jan 2019, Jan 2020), as focus is made on extracting seasonal trends.

In an embodiment, the K-means technique is used. K is an integer smaller than a number of ['None'] labelled vectors in the training feature table TFT. Then, the training feature table is filtered by retaining an equal number N of ['None'] labelled vectors per cluster and suppressing the others. In an example, where about 660 non ['None'] labelled vectors in the training feature table, N is an integer set to 20.

In an embodiment, a month sampling is further performed by additionally selecting an equal number of ['None'] labelled vectors associated with the same time period (for example, the same month) within each cluster, so as to capture seasonal changes in each cluster, ensuring these variations are also represented in the model. For example, 11 points are selected from each cluster. This results in 20 clusters * 11 points * 12 months in a year = 2640 points in total. Please note that the 11 points per cluster was chosen so that there are a total of 2640 points; this is 4 times the 660 epidemic outbreaks in the dataset. It is noteworthy that in one outbreak there can be several diseases that appeared. So the number of outbreaks can be smaller than the number of detected diseases. Through experimentation, the inventors found that selecting approximately 2500 ['None'] labelled feature vectors was optimal. This is a balance between having a lot of feature vectors to train the ML model to make it more robust, and not having too much to keep the training quite quick.

Moreover, the down sampling operation of sub-step 232 allows to avoid data imbalance between the «disease» labelled vectors (positive points) and the ['None'] labelled ones. This balance helps to improve the training of the ML model and to achieve better performance.

In another embodiment, a Kernel Density Estimation, KDE, technique may be used to estimate a probability density of data points in the ['None'] labelled vector subset, identify and remove vectors from high-density regions to retain a more diverse and representative subset of the data.

At the end of step 23, the training feature table TFT is ready for training a machine learning model. It is stored in a memory, for instance the memory M1 of system S or in a remote storage device of a distributed cloud architecture. In an embodiment, it is stored in a relational database, for example a PostgreSQL database, facilitating analysis and access for model training.

In an embodiment, the TFT comprises 3300 vectors, each containing features on distance to nearest airport, lake, trade route, river, seaport, RPT Node, and protected area; tree cover percentage; deforestation alerts; temperature; precipitation; outbreak distance; and population density. In this example, a vector comprises 13 features. A historic time period of 4 years is covered.

In an embodiment of steps 21-23, specifically designed functions may be used to read and load all the various file types into memory. This allows for seamless integration of new data sources. When a new dataset needs to be added, one only needs to build a custom feature extraction function to be used in step 22.

Referring to Figure 6A, an example of structure of a machine learning model will now be described, according to an embodiment. In this example, the machine learning model MLM is an artificial neural network (ANN) comprising a plurality of fully-connected layers. More precisely, in the example of Figure 6, the input data are feature vectors of the training feature table TFT during the learning phase. In the example, the bracketed numbers refer to the number of units in each layer. Feature vectors comprising 13 features are fed into the model to a first series of 32 dense layers. In inner layers of the series of dense layers, a ReLU (rectified linear unit) activation function is used, to map inputs to an unbounded output. The output of the first series of dense layers is applied to a dropout layer that randomly ignores 10% of all outputs from the first series of dense layer to help combat overfitting (0-1 stands for indicating that some outputs are ignored (0), whereas other are passed to the next layer (1)). The output of the dropout layer is fed into a second series of 32 dense layers. Their output is applied to a third and final series of dense layer using a softmax activation function to map inputs to output values between zero and one.

These output values can be interpreted as 'scores' for each respective disease class, where a higher score indicates a greater likelihood of the disease being present. In this example, the final layer indicates that the model is assigning an output score for 7 different diseases. Of course, this output number may vary depending on the output variables or classes used). L2 regularisation to the outputs is also added to the final series of dense layers, to smooth the outputs to further combat overfitting in the model.

The MLM structure above was chosen following extensive hyperparameter tuning. This process involved selecting the optimal combination of model structure and hyperparameters by:

systematically training the model using different structures and hyperparameters,

- evaluating the performance of each test run,
- selecting the most performant model.

During this process, a wide range of layer types (convolutional, recurrent, etc) and hyperparameter values, was experimented, ultimately finding that the above fully connected model yielded the best results.

Referring to Figure 6B, in another embodiment, two separate machine learning models are built, one disease ML model MLM1 for predicting a disease class, for example a disease type (for example Lumpy skin disease virus), and one affected taxonomic class ML model MLM2 for predicting an affected taxonomic class (for example Mammal).

It turns out that the model structure remains largely unchanged. The number of units in the final series of dense layers is all that changes, to account for the reduced number of possible labels. For example, 7 different disease class outputs (African horse sickness virus, Porcine reproductive and respiratory syndrome virus, Lumpy skin disease virus, High pathogenicity avian influenza viruses (poultry), African swine fever virus, Newcastle disease virus, and None) are provided by the first machine learning model MLM1 and 3 different taxonomic class outputs are provided by the second machine learning model MLM2

(for bird, mammal, and None classes). It is worth noting that the output dimension is 7 and 3 due to the fact the model is only trained on observations of those diseases and affected taxonomic classes. If more observations are added to the pipeline in step 21 of Figure 2, this output dimension would automatically scale upwards to account for these new observations.

In an embodiment, the machine learning models are trained using weighted categorical cross-entropy loss, which is designed to train ML models with categorical outputs. A single training cycle involves:

- splitting the input training dataset into batches of size 32 to accelerate the training process.
- passing each batch through the ML model 200 times, allowing the model to iteratively improve.

In an embodiment, the machine learning models are configured to output an estimate of probability associated with each disease class among the plurality of disease classes. During training, ground truth provided by the label data is used to evaluate an accuracy or confidence score for each predicted estimate of probability and to adjust the weights of the machine learning models while an expected accuracy threshold is not reached.

Due to the limited number samples or vectors for some diseases in the training data set TFT, overfitting poses a significant challenge for model training. To address this, a cross-validation technique is used in an embodiment, whose principle is illustrated by Figure 7. K-fold cross-validation is a machine learning technique to evaluate a ML model reliability. The training dataset is divided into a number F of separate "folds", with F a non-null integer. In the example of Figure 7, F=6. During the validation process, the ML model undergoes F training cycles. In each cycle, F-1 folds are used for training, while the remaining fold (in bold) serves as the validation set. By rotating through every fold, each data vector is used for validation exactly once. The results from all F cycles are then averaged, providing a comprehensive measure of the ML model's performance.

Once the ML model is trained, two types of output results are determined based on the model predictions:

In an embodiment, for each input vector, the output disease class associated with the highest score is classified as the 'most probable' disease.

In another embodiment, 'pseudo-risk' parameters are generated for each possible output (aside from the ['None'] disease class) as follows:

column-wise percentiles for each disease class
the percentile scores are changed to risk parameters as follows:

- Less than 90%: low risk
- Between 90% and 95%: medium risk
- Between 95% and 99%: high risk
- Above 99%: very high risk

An exception is made for diseases with very few occurrences (see Figure 5A and B); in those cases, the cutoffs are shifted to better reflect their distribution in the dataset.

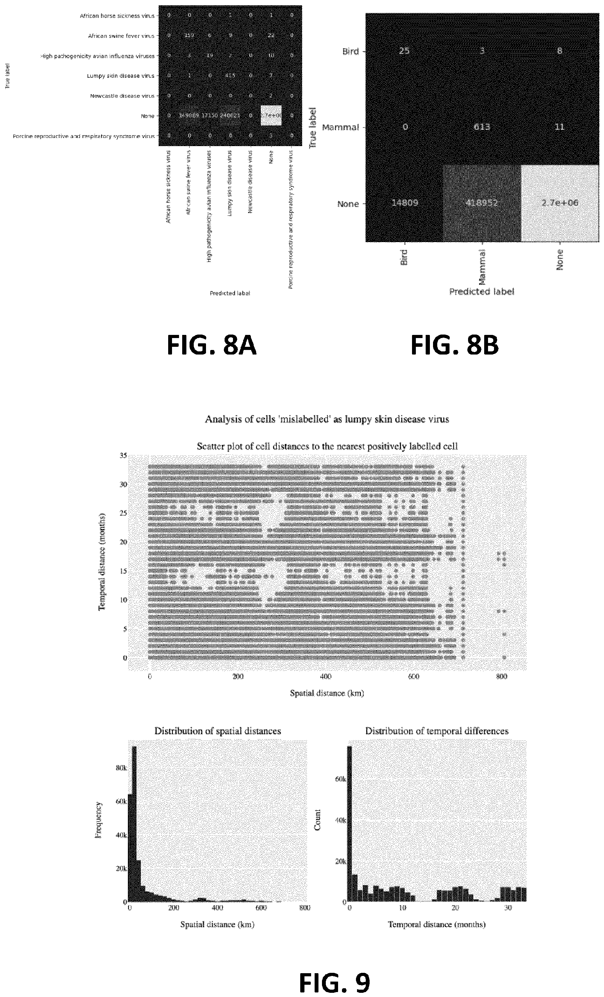

Figure 8A and 8B depict confusion matrices for both the disease and affected class models MLM1 and MLM2. They compare predicted classifications with actual classifications and serve as a useful performance measure for models. Here, entries on the leading diagonal indicate correct predictions and other entries indicate incorrect predictions. Using this confusion matrix, the following accuracy scores were calculated:

- Disease model MLM1: 87%
- Affected taxonomic class model MLM1: 86%.

It is noteworthy that there are many 'false positive' predictions, that is cells where the model has indicated it expects to see a disease in a certain region when one is actually not observed. However, this misclassification is not surprising as these cells turn out to be very close in both space and time to cells that are labelled as having a disease, leading the model to classify them similarly.

Referring to Figure 9, an exception to this pattern occurs with Lumpy Skin Disease Virus. Figure 9 shows that while false positives for this disease are still spatially close to their nearest true positive neighbour, they are temporally more spread out. This is due to there being multiple lumpy skin disease outbreaks in the dataset. Despite this, the wave-like distribution of temporal distances indicates that the ML model has successfully captured the seasonal nature of the virus.

Understanding the importance of different features in a machine learning model is crucial for interpreting the model's predictions and gaining insights into the underlying data. Back to Figure 2, in an embodiment, the training method further comprises a step 25 of determining measures quantifying a contribution of the features of the training feature table to the output information predicted by the machine learning model and a step 26 of providing the measures to a user.

In an embodiment, the determined measure are Shapley values. This allows for quantifying how much each feature contributes to the output of the model on a row-by-row basis, enhancing our insights into drivers of zoonotic outbreaks. In an embodiment, a Python's SHAP library is used to estimate these Shapley values. Referring to Figure 10, the exemplary bar chart shows that the most important factors for the MLM to predict African swine fever virus as the most probable disease class is population density (Pop), while distance from a cell to the nearest lake (Lak) is far less important.

As Shapley values are extremely computationally intensive to compute, in an embodiment, a tree-based model was trained based on the training feature table TFT to output the most probable disease class derived from the output information provided by the trained MLM (MLM or MLM1). Then, tree path dependent Shapley values are estimated, for instance using SHAP. This approach reduced the SHAP value computation time from one week (when calculated directly on the trained ML model) to a few minutes. An advantage of using a 'tree path dependent' approach is to be more 'true to the data', as discussed in this paper by Hugh Chen et. al, "True to the Model or True to the Data?" *arXiv preprint arXiv:2006.16234* (2020), https://arxiv.org/abs/2006.16234. However, it is worth noting that while SHAP values are a powerful tool for interpreting machine learning models. In an alternative, correlation and statistical analysis techniques are used to quantify the contributions of the vector features to the output predictions of the trained MLM.

A method of predicting an epidemic outbreak in a geographical area will now be described in relation to Figure 11, according to an embodiment. It may be implemented by the computer system S of Figure 1 or by any aforementioned variant of said system.

In a step 111, temporal and geospatial data relative to the geographical area GA for a current time period are obtained from a plurality of data sources, comprising raw image data captured by satellite imagery and other context related data belonging to a group comprising natural and infrastructure data, demographic data, climate data, historic disease outbreak data, and vegetation condition data. For example, the current time period is the current month.

In a step 112, the temporal and geospatial data TGSD are pre-processed. This is required to extract meaningful attributes for the targeted prediction task. In an embodiment, this step may comprise the similar sub-steps as described above for the training method in relation to Figure 3 and will not be further described. In particular, the same features are extracted as for building the training feature table TFT.

In a step 113, an input feature table IFT is built from the pre-processed temporal and geospatial data. The sub-step 231 of building a vector for each grid cell based on the extracted features is performed in a similar way to the training feature table TFT and for example as described in relation to Figure 4. This sub-step will thus not be further described. However, the vector only includes feature vectors corresponding to grid cell associated with the input time period. In addition, the sub-steps 232-234 of filtering the vectors, labelling the vectors and down-sampling the vectors are not performed.

In a step 114 the input feature table is applied to the trained ML model, for instance the ML model MLM or the two ML models ML1 and ML2 and output information related to a prediction of a disease outbreak is obtained for the plurality of cells of the grid cell and during a next time period, for instance the next month.

In an embodiment, the output information comprises an estimate of probability score of a disease outbreak for one or more classes of disease from a plurality of diseases classes that were observed to have occurred in the geographical area. In another embodiment, the output information only comprises the highest probability score and the associated class of disease.

In a step 115, prediction results RS based on the output information are provided to a user UT through a human-machine interface of the computer system. In an embodiment, 'pseudo-risk' parameters are generated from the output information (aside from the ['None'] disease class) by changing the probability score into risk parameters as follows:

- Less than 90%: low risk,
- Between 90% and 95%: medium risk,
- Between 95% and 99%: high risk,
- Above 99%: very high risk.

In an embodiment, the prediction results RS are provided to the user UT in any user friendly form, and for instance, as a graphical representation GRP. Referring to Figure 12A, the graphical representation may be a color map displayed as an overlay over a grid map, for instance comprising features like streets, landmarks, and natural elements. of the geographical area GA, for instance an OpenStreetMap® or MapBox® map. In the example of Figure 12A, the overlay comprises colored cells where outbreaks of the lumpy skin disease virus are predicted for the next month. In the example of Figure 12B, outbreaks of different diseases are indicated with different colors, one color representing a specific disease.

Each function, block, step described may be implemented in hardware, software, firmware, middleware, microcode or any suitable combination thereof. If they are implemented in software, the functions or blocks of the block diagrams and flowcharts can be implemented by computer program instructions/software codes, which can be stored or transmitted on a computer-readable medium, or loaded onto a general-purpose computer, special-purpose computer or other programmable processing device and/or system, so that the computer program instructions or software codes running on the computer or other programmable processing device create the means to implement the functions described in the present description.

In an embodiment, the computer program instructions or codebase of each function, block, step are containerised and deployed in a distributed computing architecture, for example a serverless container environment, for example, the Cloud Run environment setup provided by Google® Cloud Platform (GCP).

Figure 13 shows an example of the hardware structure of a system S of predicting an epidemic outbreak of a geographical area, according to one embodiment. In this example, the system S is integrated into an electronic apparatus EA which is configured to implement all the steps of the aforementioned methods. Alternatively, it could also implement only some of these steps.

In relation to Figure 13, the electronic apparatus EA comprises at least one processor 1110 and at least one memory 1120. The electronic apparatus EA may also comprise one or more communication interfaces. In this example, the electronic apparatus EA comprises network interfaces 1130 (for example, network interfaces for wired/wireless network access, including an Ethernet interface, a WIFI interface, etc.) connected to the processor 1110 and configured to communicate via one or more wired/wireless communication links and user interfaces 1140 (for example, a keyboard, a mouse, a display screen, etc.) connected to the processor. The electronic apparatus EA may also comprise one or more media players 1150 for reading a computer-readable storage medium (for example, a digital storage disk (CD-ROM, DVD, Blue Ray, etc.), a USB stick, etc.). The processor 1110 is connected to each of the other aforementioned components in order to control the operation thereof.

The memory 1120 may comprise a random-access memory (RAM), cache memory, non-volatile memory, backup memory (for example, programmable or flash memories), read-only memory (ROM), a hard disk drive (HDD), a solid-state drive (SSD) or any combination thereof. The ROM of the memory 1120 can be configured to store, inter alia, an operating system of the electronic apparatus EA and/or one or more computer program codes of one or more software applications. The RAM of the memory 120 can be used by the processor 1110 for temporary data storage.

The processor 1110 can be configured to store, read, load, execute and/or else process instructions stored in a computer-readable storage medium and/or in the memory 1120 so that, when the instructions are executed by the processor, the device 100 performs one or more or all of the steps of the methods disclosed herein. Means implementing a function or set of functions may correspond in this document to a software component, a hardware component or even a combination of hardware and/or software components, capable of implementing the function or set of functions, as described below for the means related.

The present description also relates to an information storage medium readable by a data processor, and comprising instructions of a program as mentioned above. The information storage medium can be any hardware means, entity or apparatus, capable of storing the instructions of the aforementioned computer program. Usable program storage media include ROM or RAM, magnetic storage media such as magnetic disks and tapes, hard disks or optically readable digital data storage media, or any combination thereof.

In some cases, the computer-readable storage medium is non-transitory. In other cases, the information storage medium may be a transient medium (for example, a carrier wave) for transmitting a signal (electromagnetic, electrical, radio or optical signal) containing program instructions. This signal can be routed via a suitable wired or wireless transmission means: electrical or optical cable, radio or infrared link, or by other means.

The above systems and methods enable the automatic prediction of epidemic outbreaks in a geographical area in the near future based on relevant features extracted from a variety of data sources and using a trained machine learning model. Experiments have proven its ability to accurately predict zoonotic disease outbreaks in the GMS region.

## Claims

1.  A computer implemented method of training a machine learning model (MLM, MLM1, MLM2) to predict an epidemic outbreak in a geographic region (GA), comprising the steps of:

    - obtaining (21) temporal and geospatial data (TGSD) related to said geographical area over a series of successive time periods from a plurality of data sources (DS1-DSN), comprising satellite imagery, said temporal and geospatial data belonging to a group comprising natural and infrastructure data, demographic data, climate data, historic disease outbreak data, and vegetation condition data;
    - building (23) a training feature table (TFT), said training feature table comprising a plurality of feature vectors (V) respectively associated with a plurality of cells of a cell grid (SPT-GRD) mapped across the geographical area and over the series of successive time periods, one of said feature vectors comprising features extracted from said temporal and geospatial data intersecting the associated cell and being further associated with label data (LBD) indicating at least if an epidemic outbreak has occurred or not during a time period of the associated cell; and
    - training (24) the at least one machine learning model to predict output information related to an epidemic outbreak in the geographical area for a next time period from feature vectors of the training feature table associated with a current time period.

2. The method according to the preceding claim, further comprising the step of:

- preprocessing (23) the temporal and geospatial data comprising forming (222) the cell grid across the geographical area and over the series of successive time periods and extracting (223) the data features from the temporal and geospatial data intersecting the plurality of cells.

3. The method according to the preceding claim, wherein the geospatial data comprises locations of natural and infrastructure objects of interest in the geographical area and extracting data features (223) comprises calculating a minimal spatial distance between the cell and nearby objects of interest and storing the calculated minimal spatial distances in the feature vector of the cell.

4. The method according to anyone of claims 2 to 3, wherein the temporal and geospatial data comprises disease outbreak reports in the geographical area and extracting data features (223) comprises calculating an inverse distance metrics between the cell and nearby cells where a previous epidemic outbreak was observed to have occurred within a given time range and storing the inverse distance metrics in the feature vector of the cell.

5. The method according to anyone of claims 2 to 4, wherein the temporal and geospatial data comprises locations of vegetation condition alerts associated with a date of disturbance and a confidence level and extracting data features (223) comprises calculating a number of high-confidence vegetation condition alerts having occurred within the cell and storing the number of high-confidence vegetation condition alerts in the feature vector.

6. The method according to anyone of the preceding claims, wherein the label data comprises a disease class among a plurality of disease classes comprising a ['None'] disease class, the building step (23) comprises selecting feature vectors having a label class of disease equal to the ['None'] class, or ['None'] labelled feature vectors, clustering (233) the ['None'] labelled feature vectors into a number K of clusters based on a minimum spatial distance criteria, K being an integer smaller than a number of ['None'] labelled feature vectors in said training feature table, and filtering said training feature table by retaining a same number N of ['None'] labelled feature vectors per cluster.

7. The method according to anyone of the preceding claims, further comprising the steps of

- determining (25) measures quantifying a contribution of the features of the training feature table to the output information predicted by the machine learning model and
- providing (26) the measures to a user.

8. The method according to anyone of the preceding claims, further comprising the steps of:

- detecting a new disease observed to have occurred in the geographical area from one or more of the plurality of data sources,
- updating the label data and building an updated training feature table comprising feature vectors labelled with the updated labelled data; and
- retraining the at least one machine learning model based on the updated training feature table.

9. A computer implemented method of predicting an epidemic outbreak in a geographical area, including the following steps, performed by a computer system:

- obtaining (111) temporal and geospatial data (TGSD) relative to said geographical area (GA) for a current time period from a plurality of data sources, comprising satellite imagery, said temporal and geospatial data (TGSD) belonging to a group comprising natural and infrastructure data, demographic data, climate data, historic animal disease outbreak data, and vegetation condition data;
- building (113) an input feature table (IFT), said input feature table comprising a plurality of vectors respectively associated with a plurality of cells of a cell grid mapped across the geographical area for the current time period, one of said feature vectors comprising features extracted from said temporal and geospatial data intersecting with the associated cell during the current time period of the associated cell; and
- predicting (114), based on the input feature table (IFT) and by using at least one trained machine learning model (MLM, MLM1; MLM2), output information (EOI) indicating at least if an epidemic outbreak is likely to occur or not in the cell during a next time period; and

- providing (115) prediction results based on the output information to a user (UT) through a human-machine interface (UI) of the computer system (S).

10. The method according to the preceding claim, further comprising the step of:

- preprocessing (112) the temporal and geospatial data comprising forming (222) the cell grid across the geographical area and over the series of successive time periods and extracting the data features from the geospatial data intersecting the plurality of cells.

11. The method according to anyone of claims 9 and 10, wherein the predicting step (114) is carried out by a first and a second trained machine learning models (MLM1, MLM2, based on the feature table, the first trained machine learning model being configured to output, for one cell of the cell grid, an estimate of a highest probable class of disease among a plurality of classes of diseases, and the second trained machine learning model being configured to output a taxonomic class of animal predicted to be affected by the class of disease.

12. A computer system (S) of predicting an epidemic outbreak in a geographical area, said computer system comprising:

- a communication component (GET) configured to obtain temporal and geospatial data (TGSD) relative to said geographical area (GA) for a current time period from a plurality of data sources, comprising satellite imagery, said temporal and geospatial data belonging to a group comprising nature and infrastructure data, demographic data, climate data, historic animal disease outbreak data, and vegetation condition data;
- a building component (BLD) configured to build an input feature table (IFT), said input feature table comprising a plurality of vectors respectively associated with a plurality of cells of a cell grid mapped across the geographical area for the current time period, one of said feature vectors comprising features extracted from said temporal and geospatial data intersecting with the associated cell during the current time period of the associated cell;
- a predicting (PRD) component configured to predict, based on the input feature table (IFT) and by using at least one trained machine learning model (MLM, MLM1; MLM2), output information (EOI) indicating at least if an epidemic outbreak is likely to occur or not in the cell during a next time period; and
- a human-machine interface (UI) configured to provide prediction results (RS) based on the output information to a user (UT).

13. The computer system (S) according to the preceding claim, further comprising a training component (TRN) configured to train the machine learning model to predict output information related to an epidemic outbreak in a geographic region, and comprising:

- obtaining temporal and geospatial data (TGSD) related to said geographical area over a series of successive time periods from a plurality of data sources (DS1-DSN), comprising satellite imagery, said temporal and geospatial data belonging to a group comprising nature and infrastructure data, demographic data, climate data, historic disease outbreak data, and vegetation condition data;
- building a training feature table (TFT), said training feature table comprising a plurality of feature vectors (V), one of said plurality of feature vectors being associated with one cell of a plurality of cells of a cell grid (SPT-GRD) mapped across the geographical area and over the series of successive time periods, said vector comprising features extracted from said temporal and geospatial data and label data (LBD) indicating at least if an epidemic outbreak has occurred or not during a time period of the cell;
- training the at least one machine learning model to predict output information related to an epidemic outbreak in the geographical area for a next time period from vectors of the training feature table associated with a current time period.

14. The computer system according to anyone of claims 12 and 13, wherein the means comprises

- at least one processor; and
- at least one memory including computer program code, the at least one memory and computer program code configured to, with the at least one processor, cause the computer system to carry out the steps of the method of any of claims 1 to 11.

15. A non-transitory computer-readable medium comprising program instructions stored thereon for causing a computer to perform a method according to any one of claims 1-11.

**FIG. 1**

**FIG. 2**

**FIG. 3**

**FIG. 4A**

**FIG. 4B**

**FIG. 4C**

**FIG. 4D**

Disease counts before and after filtering

**FIG. 5A**

**FIG. 5B**

**FIG. 6A**

**FIG. 6B**

| All data | | | | | |
|---|---|---|---|---|---|

| Training data | | | | Test data | |
|---|---|---|---|---|---|

| Fold 1 | Fold 2 | Fold 3 | Fold 4 | Fold 5 | Fold 6 |
|---|---|---|---|---|---|
| Fold 1 | Fold 2 | Fold 3 | Fold 4 | Fold 5 | Fold 6 |
| Fold 1 | Fold 2 | Fold 3 | Fold 4 | Fold 5 | Fold 6 |
| Fold 1 | Fold 2 | Fold 3 | Fold 4 | Fold 5 | Fold 6 |
| Fold 1 | Fold 2 | Fold 3 | Fold 4 | Fold 5 | Fold 6 |
| Fold 1 | Fold 2 | Fold 3 | Fold 4 | Fold 5 | Fold 6 |

**FIG. 7**

FIG. 8A

FIG. 8B

Analysis of cells 'mislabelled' as lumpy skin disease virus

Scatter plot of cell distances to the nearest positively labelled cell

Distribution of spatial distances

Distribution of temporal differences

FIG. 9

**FIG. 10**

**FIG. 11**

**FIG. 12A**

**FIG. 12B**

**FIG. 13**

Europäisches
Patentamt

European
Patent Office

Office européen
des brevets

# EUROPEAN SEARCH REPORT

**Application Number**

EP 24 30 7202

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/384055 A1 (GOPALAKRISHNAN VISHRAWAS [US] ET AL) 1 December 2022 (2022-12-01) * paragraphs [0001], [0007], [0027], [0032] - [0035], [0040], [0065], [0072], [0074], [0088], [0094], [0107], [0182]; claim 11 * | 1-15 | INV.<br>G16H50/70<br>G16H50/80 |
| | ----- | | |
| X | US 2022/199266 A1 (ACHIN JEREMY [US] ET AL) 23 June 2022 (2022-06-23) * paragraphs [0001], [0007], [0012], [0028], [0085], [0172], [0177], [0203] - [0204] * | 1-15 | |
| | ----- | | |
| X | US 2024/395420 A1 (ALEXANDER CALEB [US] ET AL) 28 November 2024 (2024-11-28) * paragraphs [0016], [0031], [0066] * | 1-15 | |
| | ----- | | |
| A | US 2009/082997 A1 (TOKMAN MICHAEL G [US] ET AL) 26 March 2009 (2009-03-26) * the whole document * | 1-15 | **TECHNICAL FIELDS SEARCHED (IPC)**<br><br>G16H |
| | ----- | | |

The present search report has been drawn up for all claims

| Place of search | Date of completion of the search | Examiner |
|---|---|---|
| Munich | 2 June 2025 | Huber, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 24 30 7202

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2025

| Patent document cited in search report | | Publication date | Patent family member(s) | | Publication date |
|---|---|---|---|---|---|
| US 2022384055 A1 | | 01-12-2022 | NONE | | |
| US 2022199266 A1 | | 23-06-2022 | NONE | | |
| US 2024395420 A1 | | 28-11-2024 | NONE | | |
| US 2009082997 A1 | | 26-03-2009 | EP | 1964014 A1 | 03-09-2008 |
| | | | US | 2009082997 A1 | 26-03-2009 |
| | | | WO | 2007076426 A1 | 05-07-2007 |

EPO FORM P0459

For more details about this annex : see Official Journal of the European Patent Office, No. 12/82